# EUROPEAN PATENT SPECIFICATION

(11) **EP 0 368 156 B1**
(45) Date of publication and mention of the grant of the patent: **01.09.1993**
(21) Application number: 89120353.1
(22) Date of filing: 03.11.1989
(51) Int. Cl.: C07C 47/228, C07C 47/232, A61K 7/46

(54) **Aromatic-aliphatic aldehydes**
Araliphatische Aldehyde
Aldéhydes aromatiques-aliphatiques

(30) Priority: 10.11.1988 US 269516
(43) Date of publication of application: 16.05.1990
(73) Proprietor: L. GIVAUDAN & CIE Société Anonyme, CH-1214 Vernier-Genève (CH)
(72) Inventor: Chalk, Alan J., Fayson Lake Kinnelon N.J. 07405 (US)
(74) Representative: Urech, Peter, Dr.

(56) References cited:
- EP-A- 0 045 571
- DE-A- 2 340 812
- DE-B- 1 145 161

## Description

This invention concerns the novel compounds 3-(3-propan-2-ylphenyl)butanal (Ia) and 3-(3-propen-2-ylphenyl)butanal (Ib) which can be represented by the formula 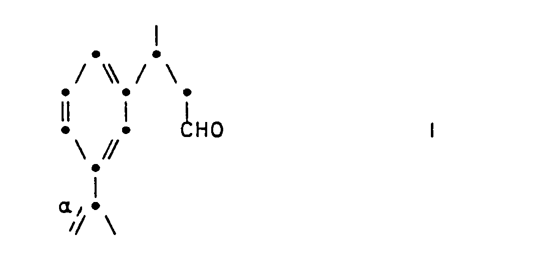
wherein the dotted line, α, represents an optional bond.

The compounds of the present invention have odors which can be described as fresh, floral, fruity, melon having qualities found in lily-of-the-valley (muguet) and linden blossom. They are useful as odorants and may be used in perfumes, colognes, soaps, detergents, cosmetics and other toilet goods.

The novel compounds of the present invention can be prepared according to Scheme I. 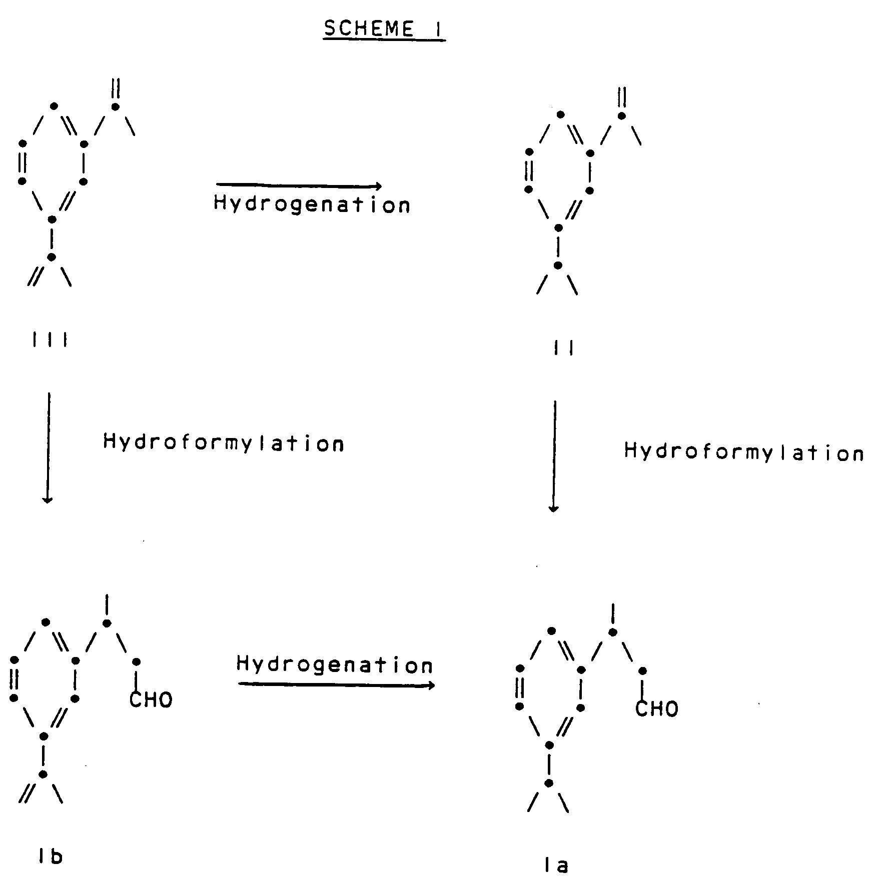
As illustrated in Scheme I, the novel compounds of this invention can be prepared from the readily available m-diisopropenylbenzene, III. (The starting material III is commercially available or may be prepared by known methods, e.g., see H. A. Colvin and J. Muse, Chemtech 15, (1986) 500-4.) The 3-(3-Propen-2-ylphenyl)butanal, Ib, can be prepared by hydroformylation of the starting material, III, as shown in Scheme I and illustrated by Example I. It is preferred to prepare the compound Ia, via the hydrogenation of Ib, also shown in Scheme I and illustrated by Example II. An alternate synthesis of Ia involves reducing one of the olefinic bonds of III to form II and then converting II to Ia via hydroformylation.

The olefinic hydrogenation depicted above may be carried out in a manner known per se for hydrogenating olefinic double bonds in the presence of phenyl radicals and, in the case of Ib to Ia, in the presence of carbonyls also. Typical catalysts for such conversions are palladium on charcoal or Raney nickel, etc. Typical pressure range from atmospheric to 4,08 bar (60 psi)and typical temperatures range from 0°C to 100°C.

Because the separation of II from III is difficult the conversion of III to II and then to Ia is not preferred: This process is best achieved by stopping the hydrogenation of III after about 30% or 40% hydrogen uptake and then separating the product II from III by a very efficient distillation.

The hydroformylations depicted above can be effected in a manner known per se for the preparation of aldehydes from α-substituted styrenes using CO, H₂ and an appropriate catalyst such as rhodium in any of its suitable forms. Suitable catalysts are for e.g. rhodium phosphine complexes such as HRh(CO)(PPh₃)₃, ClRh(CO)(PPh₃)₂ etc. or a compound of rhodium such as RHCl₃, Rh acetonylacetonate, etc. modified by phosphine ligands. A suitable pressure range is 1,02-204 bar (15-3000 psi) but preferably 6,8-34 bar (100-500 psi). Temperatures are in the range of 50°-200°C but preferably 100°-150°C.

Selective hydroformylations of the type similar to the conversion of III to Ib are best carried out by breaking off the reaction after ca. 30-40% conversion, separating the product by distillation and recycling the starting material.

Compounds Ia and Ib have fresh, floral, fruity, melon odors with qualities of lily-of-the-valley and linden blossom which make them particularly valuable in perfumery. They are most useful in providing a natural floral quality and lift to fragrance bases. The fresh quality which may be described as ozone-like, is more predominant in compound Ib, whereas in compound Ia, the floral character is more predominant.

Compound Ia, which has a fine, floral, melon odor is the more preferred compound of the invention. It has those qualities found in lily-of-the-valley and linden blossom to a greater degree than Ib. The compound is powerful and diffusive and can be used widely for floral effects and to give long lasting fresh, green topnotes. It is useful for lilac, rose, lily, peony, magnolia, orange blossom and other related fragrances.

Compound Ib has a floral stemmy-green, melon odor with a clean, ozone character. Its odor notes make Ib particularly useful in citrus, green-herbal and floral aldehyde fragrance compositions.

The compounds can be used in fragrances e.g. in the ratio of about 1 to 200 parts per thousand of the odorant composition containing the compound. A range of 50 to 100 parts is preferred for most compositions. Larger quantities, e.g., 200-900 parts can be used to achieve special effects.

The odorant compositions containing the compounds of this invention can be used as odorant bases for the preparation of, e.g. perfumes and toilet waters by adding the usual alcoholic and aqueous diluents thereto; approximately 15-20 percent by weight of base would be used for the former and approximately 3-5 percent by weight would be used for the latter.

Similarly, the base compositions can be used to odorize soaps, detergents, cosmetics, or the like. In these instances a basic concentration of from about 0.5 to about 2 percent by weight can be used.

The following Examples are provided to illustrate further the practice of the present invention. They are for purposes of preferred embodiments only and should not be construed as limiting.

### EXAMPLE I

Preparation of 3-(3-propen-2-ylphenyl)butanal, Ib.

m-Diisopropenylbenzene (237g), 0.2g butylated hydroxytoluene, 0.1g NaHCO₃, 0.0918g RhHCO(PPh₃)₃ and 20g triphenylphosphine were heated under a pressure of 17 bar (250 psi) hydrogen and 17 bar (250 psi) carbon monoxide at 110°C for five hours and then at 140°C for six hours. The crude reaction product was distilled to give 215g of a liquid which analyzed as 49.4% starting material, 40.8% Ib, 1.9% 2-(3-propen-ylphenyl)-2-methylpropanal and 4.9% 1,3-di-(1-formylpropan-2-yl)benzene. The constituents were readily separated by fractional distillation to give pure Ib, b.p 98°C/0,133·10⁻³ bar (0.1 mm Hg). The starting material may be recycled to give further product in an overall yield of 71.0% Ib. IR and NMR spectra were compatible with the assigned structure. Odor: ozone, stemmy-green floral, melon. Odor value: 35,514; Odor threshold value: 0.7ng/L. (see below)

### EXAMPLE II

### Preparation of 3-(3-propan-2-ylphenyl)butanal, Ia.

3-(3-Propen-2-ylphenyl)butanal, Ib, (100 g) was hydrogenated over palladium on charcoal (5% wet, 1 gram) in 150 ml ethanol for 2.5 hours at 3,4 bar (50 psi) hydrogen using a Parr-shaker apparatus. The product mixture was decanted, filtered and the ethanol removed on a rotary evaporator to give 97 grams of a mixture which analyzed as 95.8% Ia, and 2% Ib. The mixture was distilled to give 78.1g pure Ia, b.p. 84°C/0,399·10⁻³ bar (0.3 mm Hg). Yield: 78.0% Ia. IR and NMR spectra were compatable with the assigned structure. Odor: fresh, floral, melon having qualities found in lily-of-the-valley (muguet) and linden blossom. Odor value: 981,483; Odor threshold value: 0.07 ng/L.

These values are surprising since the odor threshold of the positional isomer known as cyclamen aldehyde, a widely used odorant (see Arctander, Perfume and Flavour Chemicals I, item 758; Montclair, N.J. 1969) 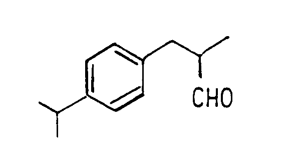
is 3.0 ng/L, i.e., cyclamenaldehyde is only detected at a concentration 43 times higher than that of I. The odor value of I is higher than that of cyclamenaldehyde (18,467) by a similar large factor. Furthermore, DT-OS 2340812 describes, inter alia, the compound of the formula 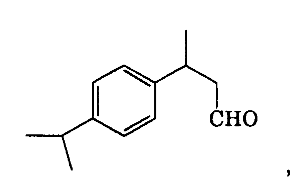
said compound is said to exhibit agreeable odorant properties and can be used for the manufacture of a number of essential oils.

### EXAMPLE III

### Muguet Fragrance with 15% Ia for soap bar

Without Ia the composition was found to be of moderate intensity and somwhat unblended in that the rose notes dominated. The addition of Ia improved the floral character of the composition by contributing a watery or dewy quality, an effect which changed the impression from rose to lily-of-the-valley making the composition more natural and reminiscent of the flower. Ia made the odor effect markedly stronger.

| Component | Parts |
|---|---|
| Phenylethyl alcohol | 200 |
| Citronellol | 300 |
| Geraniol | 150 |
| Benzyl Acetate Extra | 60 |
| Nonane-1,3-diol acetate | 10 |
| Amyl Cinnamic Aldehyde | 75 |
| Aldehyde C-14 (myristic aldehyde) | 4 |
| Heliotropin | 50 |
| Compound Ia | 150 |
| Dipropylene glycol | 1 |
| | 1̅,̅0̅0̅0̅ |

### EXAMPLE IV

### Citrus Fragrance with 10% Ia for cleaning products

Without Ia the composition was found to be of moderate intensity. With the addition of Ia the intensity was enhanced and a tartness was added which makes the citrus fragrance fresher, stronger and a more desirable citrusy odor type.

| Component | Parts |
|---|---|
| Bergamot Synthetic | 200 |
| Citron Synthetic | 400 |
| Geranyl Nitrile | 5 |
| Geraniol | 100 |
| Litsea Cubeba oil | 10 |
| Cyclohexanol, 4-(1,1-dimethylethyl)-, acetate | 50 |
| Anthranilic acid, N-[3-(1,1-dimethylethyl)phenyl]-2-methylpropylidene-, methyl ester | 5 |
| Eucalyptol | 10 |
| Compound Ia | 100 |
| Dipropylene glycol | 120 |
| | 1̅,̅0̅0̅0̅ |

### EXAMPLE V

### Rose Musk fragrance with 2% Ia

Without Ia the woody and musky notes stood out from the composition. The addition of Ia added a strong floral quality to the fragrance which complimented the woody and musky odors. The fragrance with Ia is brighter, fresher, and stronger than the fragrance without Ia.

| Components | Parts |
|---|---|
| Geranium Bourbon | 15 |
| Linalool | 50 |
| Rhodinol Pure | 50 |
| Methyl ionone | 60 |
| 1-(2,6,6-Trimethyl-2-cyclohexen-1-yl)-hepta-1,6-dien-3-one | 5 |
| Methyl Cedryl Ketone | 150 |
| Heliotropin | 40 |
| Coumarin | 70 |
| 4-t-Butyl-3,5-dinitro-2,4-dimethylacetophenone | 50 |
| Cinammic Alcohol | 5 |
| Vanillin | 10 |
| Sandela® (Givaudan) (isocamphylcyclohexanols) | 150 |
| Crysolide® (Givaudan) (4-Acetyl-6-t-butyl-1,1-dimethylindane) | 100 |
| Synthetic Rose Base | 140 |
| Synthetic Civet Base | 1 |
| Compound Ia | 20 |
| Dipropylene glycol | 84 |
| | 1̅,̅0̅0̅0̅ |

## Claims (Claims for the following Contracting State(s): AT, BE, CH, DE, FR, GB, IT, LI, NL)

1. A compound of the formula 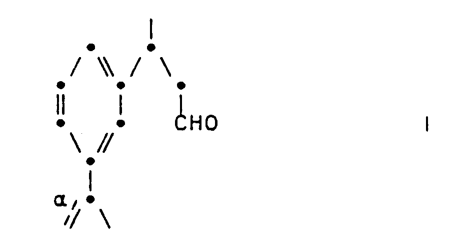 wherein the dotted line α represents an optional bond

2. 3-(3-propan-2-ylphenyl)butanal.

3. 3-(3-Propen-2-ylphenyl)butanal.

4. A fragrance composition comprising an olfactorily effective amount of a compound of the formula 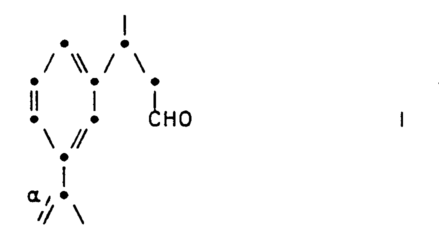 wherein the dotted line α represents an optional bond.

5. The composition according to claim 4 wherein the compound is 3-(3-Propan-2-ylphenyl)butanal.

6. The composition according to claim 4 wherein the compound is 3-(3-propen-2-ylphenyl)butanal.

7. Process for the manufacture of the compounds of formula 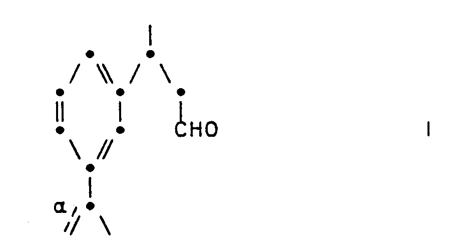 wherein the dotted line, α, represents an optionyl bond,
comprising hydroformylating a compound of formula 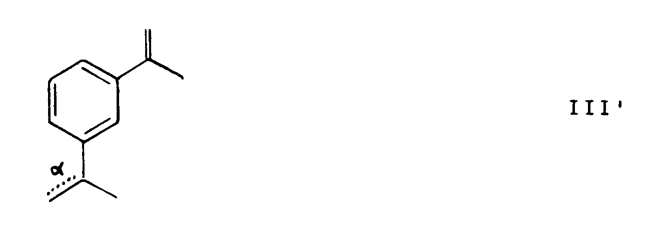 wherein α is as above,
and, if desired, hydrogenating any double bond being present in position α.

8. The use of the compounds of formula I of claim 1 as odorants.

## Claims (Claims for the following Contracting State(s): ES)

1. A fragrance composition comprising an olfactorily effective amount of a compound of the formula 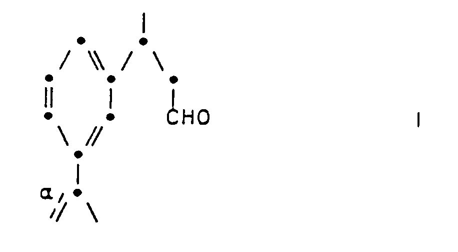 wherein the dotted line α represents an optional bond.

2. The composition according to claim 1 wherein the compound is 3-(3-propan-2-ylphenyl)butanal.

3. The composition according to claim 1 wherein the compound is 3-(3-propen-2-ylphenyl)butanal.

4. Process for the manufacture of the compounds of formula 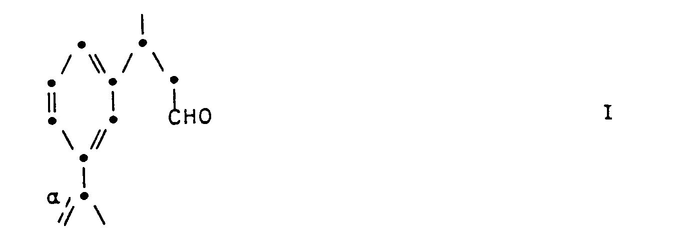 wherein the dotted line, α, represents an optionyl bond,
comprising hydroformylating a compound of formula 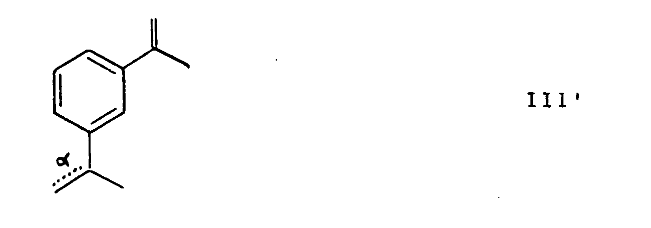 wherein α is as above,
and, if desired, hydrogenating any double bond being present in position α.

5. The use of the compounds of formula I of claim 1 as odorants.

## Patentansprüche (Patentansprüche für folgende(n) Vertragsstaat(en): AT, BE, CH, DE, FR, GB, IT, LI, NL)

1. Eine Verbindung der Formel 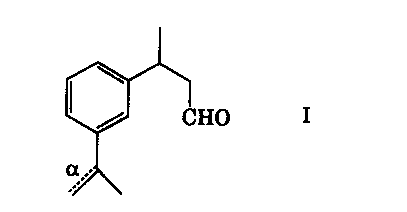 worin die gestrichelte Linie α eine fakultative Bindung bedeutet.

2. 3-(3-Propan-2-ylphenyl)butanal.

3. 3-(3-Propen-2-ylphenyl)butanal.

4. Riechstoffkomposition, gekennzeichnet durch einen olfaktorisch wirksamen Gehalt an einer Verbindung der Formel 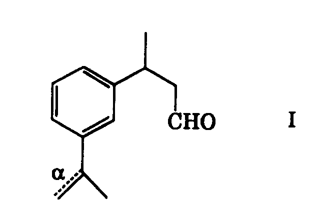 worin die gestrichelte Linie α eine fakultative Bindung bedeutet.

5. Komposition gemäss Anspruch 4, worin die Verbindung 3-(3-Propan-2-ylphenyl)butanal ist.

6. Komposition gemäss Anspruch 4, worin die Verbindung 3-(3-Propen-2-ylphenyl)butanal ist.

7. Verfahren zur Herstellung der Verbindungen der Formel 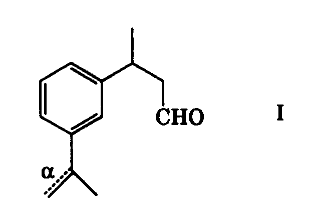 worin die gestrichelte Linie α eine fakultative Bindung bedeutet. dadurch gekennzeichnet, dass man eine Verbindung der Formel 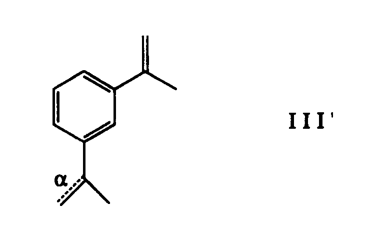 worin α obige Bedeutung besitzt, hydroformyliert, und, gegebenenfalls eine allfällig vorhandene Doppelbindung in Stellung α hydriert.

8. Verwendung der Verbindungen der Formel I des Anspruchs 1 als Riechstoffe.

## Patentansprüche (Patentansprüche für folgende(n) Vertragsstaat(en): ES)

1. Riechstoffkomposition, gekennzeichnet durch einen olfaktorisch wirksamen Gehalt an einer Verbindung der Formel 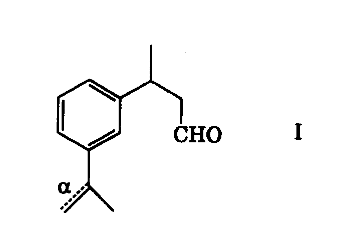 worin die gestrichelte Linie α eine fakultative Bindung bedeutet.

2. Komposition gemäss Anspruch 1, worin die Verbindung 3-(3-Propan-2-ylphenyl)butanal ist.

3. Komposition gemäss Anspruch 1, worin die Verbindung 3-(3-Propen-2-ylphenyl)butanal ist.

4. Verfahren zur Herstellung der Verbindungen der Formel 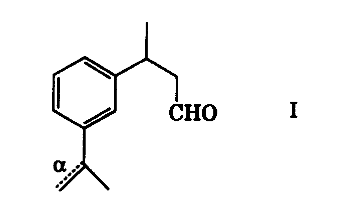 worin die gestrichelte Linie α eine fakultative Bindung bedeutet, dadurch gekennzeichnet, dass man eine Verbindung der Formel 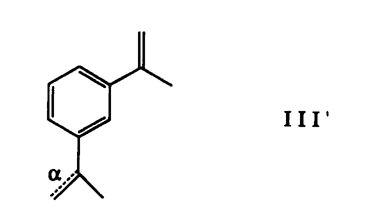 worin α obige Bedeutung besitzt,
hydroformyliert, und, gegebenenfalls eine allfällig vorhandene Doppelbindung in Stellung α hydriert.

5. Verwendung der Verbindungen der Formel I des Anspruchs 1 als Riechstoffe.

## Revendications (Revendications pour l'(les) Etat(s) contractant(s) suivant(s): AT, BE, CH, DE, FR, GB, IT, LI, NL)

1. Composé de formule 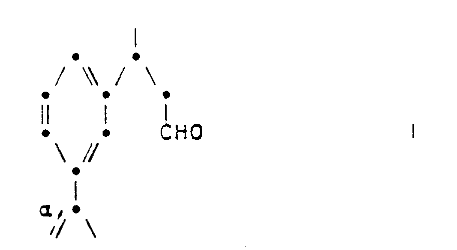 dans laquelle le pointillé α représente une liaison facultative.

2. 3-(3-propane-2-ylphényl)butanal.

3. 3-(3-propène-2-ylphényl)butanal.

4. Composition de parfum comprenant une quantité efficace pour l'olfaction d'un composé de formule 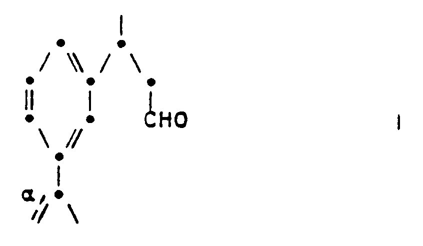 dans laquelle le pointillé α représente une liaison facultative.

5. Composition selon la revendication 4, dans laquelle le composé est le 3-(3-propane-2-ylphényl)butanal.

6. Composition selon la revendication 4, dans laquelle le composé est le 3-(3-propène-2-ylphényl)butanal.

7. Procédé pour la préparation des composés de formule 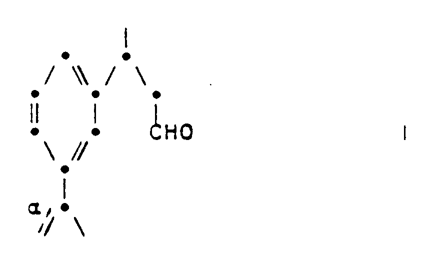 dans laquelle le pointillé α représente une liaison facultative, comprenant l'hydroformylation d'un composé de formule 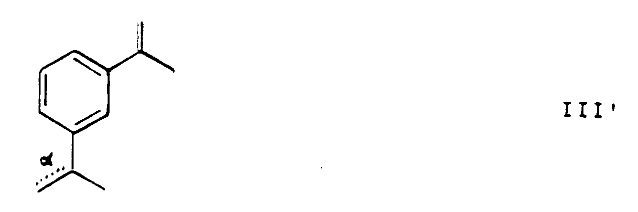 dans laquelle α est défini comme ci-dessus, et, si on le désire, l'hydrogénation d'une double liaison présente en position α.

8. Utilisation des composés de formule I de la revendication 1 comme odorants.

## Revendications (Revendications pour l'(les) Etat(s) contractant(s) suivant(s): ES)

1. Composition de parfum comprenant une quantité efficace pour l'olfaction d'un composé de formule 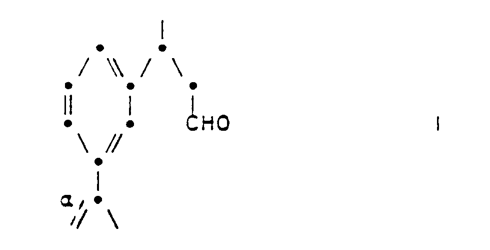 dans laquelle le pointillé α représente une liaison facultative.

2. Composition selon la revendication 1, dans laquelle le composé est le 3-(3-propane-2-ylphényl)butanal.

3. Composition selon la revendication 1, dans laquelle le composé est le 3-(3-propène-2-ylphényl)butanal.

4. Procédé pour la préparation des composés de formule 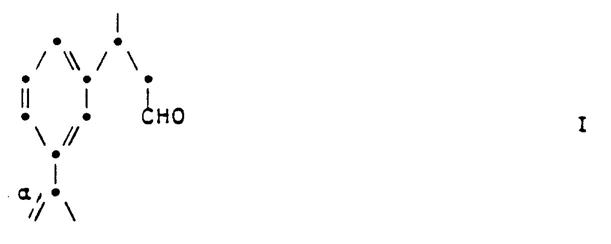 dans laquelle le pointillé α représente une liaison facultative, comprenant l'hydroformylation d'un composé de formule 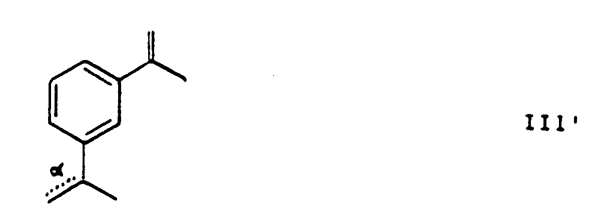 dans laquelle α est défini comme ci-dessus, et, si on le désire, l'hydrogénation d'une double liaison présente en position α.

5. Utilisation des composés de formule I de la revendication 1 comme odorants.
